(19) Europäisches Patentamt
European Patent Office
Office européen des brevets

(11) Publication number: **0 265 162 B1**

(12) # EUROPEAN PATENT SPECIFICATION

(45) Date of publication of patent specification: **06.03.91**

(51) Int. Cl.⁵: **C07D 263/28**, A01N 43/76

(21) Application number: **87309069.0**

(22) Date of filing: **14.10.87**

(54) **Iminooxazolidines, process of preparation and method of use.**

(30) Priority: **17.10.86 US 920014**
**17.10.86 US 920345**

(43) Date of publication of application:
**27.04.88 Bulletin 88/17**

(45) Publication of the grant of the patent:
**06.03.91 Bulletin 91/10**

(84) Designated Contracting States:
**AT BE CH DE ES FR GB GR IT LI NL SE**

(56) References cited:
**DE-A- 1 963 192**

**Chemical Abstracts, vol. 75, no. 25, December 20, 1971, Columbus, Ohio, USA; BELZECKI, C; PEKSA, S "Reactions of epoxy compounds with guanidine derivatives. II. Reaction of styrene oxide with alkyl- and alkyl(aryl)guanidines" page 316, column 2, abstract-no. 151 714k**

(73) Proprietor: **STAUFFER CHEMICAL COMPANY**

**Westport Connecticut 06881(US)**

(72) Inventor: **Felix, Raymond Anthony**
**5949 Ralston Avenue**
**Richmond California 94805(GB)**

(74) Representative: **Froud, Clive et al**
**ELKINGTON AND FIFE Beacon House 113 Kingsway**
**London WC2B 6PP(GB)**

**EP 0 265 162 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

Chemical Abstracts, vol. 77, no. 13, September 25, 1972, Columbus, Ohio, USA; BEACHELL, H.C.; CHU-PHAM-NGOC-SON; NGUYEN-HUU-TIHN "Pyrolysis of N-((beta-(phenylcarbamoyl)ethyl))-N,N'-diphenylurea. Synthesis and properties of the decomposition product, 2-(phenylimino)-3-phenyloxazolidine and its analogs" page 451, column 1, abstract-no. 88 373x

Chemical Abstracts, vol. 71, no. 21, November 24, 1969, Columbus, Ohio, USA; OHASHI, TAKASHI; MITSUNOBU, TAKEHIRO; MUKAIYAMA, MITSUAKI; KUBOTA, NAOTAKE "Oxazolidines and imidazolidinones" page 354, column 2, abstract-no. 101 855d & Japan. 69 21,095

Chemical Abstracts, vol 105, no. 1, July 7, 1986, Columbus, Ohio, USA; SHIBATA, IKUYA; BABA, AKIO; IWASAKI, HIROYUKI; MATSUDA, HARUO "Cycloaddition reaction of heterocumulenes with oxiranes catalyzed by organotin iodide-Lewis base complex" page 602, column 1, abstract-no. 6 435d

## Description

The present invention relates to certain iminooxazolidine herbicide compounds, compositions, process of preparation and methods of use.

Some structurally-similar compounds are known, (see Beachell, H. C., J. Org. Chem., 37 (3), 1972, 422-5), but are not herbicidal.

Herbicides have been used for many years by farmers, commercial agricultural companies and other industries in order to eliminate weed pests and thereby increase crop yields of such staple crops as corn, soybeans, rice and the like.

There are a number of different types of herbicides presently sold commercially, and these fall into two general categories. The categories are pre-emergence and post-emergence herbicides. The pre-emergence herbicides are normally incorporated into or applied to the soil prior to the emergence of the weed plants from the soil, and the post-emergence herbicides are normally applied to plant surfaces after emergence of the weeds or other unwanted plants from the soil. Some herbicides are effective both as pre- and post-emergence herbicides. The iminooxazolidines of this invention fall into that category.

It has now been discovered that certain iminooxazolidines have good herbicidal and plant growth regulating activity, when applied either pre- or post-emergence and used against annual and perennial grasses and broadleaf weeds.

As used herein, the term "herbicide" means a compound or composition which adversely controls or modifies the growth of plants. By the term "herbicidally effective amount" is meant any amount of such compound or composition which causes an adverse modifying effect upon the growth of plants. By "plants" is meant germinant seeds, emerging seedlings and established and established vegetation, including roots and above-ground portions. Such controlling or modifying effects include all deviations from natural development, such as killing, retardation, defoliation, desiccation, regulation, stunting, tillering, leaf burn, dwarfing, and the like.

The compounds of this invention are iminooxazolidines having the formula

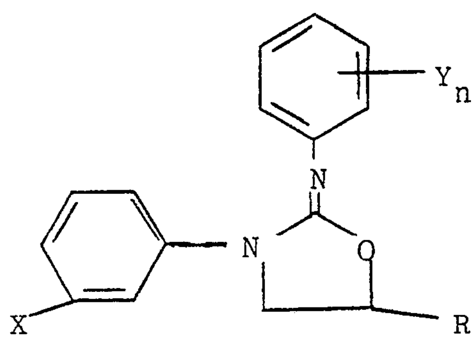

wherein

x is selected from the group consisting of cyano, halogen, alkyl, alkylthio, haloalkyl, haloalkylthio, alkylsulfenyl, alkoxy, carboalkoxy and haloalkoxy wherein the alkyl groups have from 1 to 5 carbon atoms;

y is selected from the group consisting of hydrogen, cyano, halogen, alkyl, alkylthio, haloalkyl, haloalkylthio, alkylsulfenyl, alkoxy, carboalkoxy and haloalkoxy wherein the alkyl groups have from 1 to 5 carbon atoms;

n is the integer 1 or 2; and

R is hydrogen or a lower alkyl group having from 1 to 3 carbon atoms, preferably an ethyl group, and herbicidally effective salts thereof.

The compositions of the invention comprise the aforementioned herbicide compounds, along with inert additives, as set forth more fully hereinbelow.

The method of the invention comprises the application to the locus where control is desired either the compound(s) or composition containing the compound(s) described herein.

Representative compounds falling within the scope of the formula as set forth above include:

2-[N-(3-trifluoromethyl)phenyl]imino-3-(3-trifluoromethyl)phenyl5-ethyl oxazolidine
2-[N-(3-trifluoromethyl)phenyl]imino-3-(3-cyano)phenyl-5-ethyl oxazolidine
2-[N-(4-chloro)phenyl]imino-3-(3-cyano)phenyl-5-ethyl oxazolidine
2-[N-(4-chloro)phenyl]imino-3-(3-trifluoromethyl)phenyl-5-ethyl oxazolidine
2-[N-(4-chloro)phenyl]imino-3-(4-chloro)phenyl-5-ethyl oxazolidine
2-[N-(3-trifluoromethyl)phenyl]imino-3-(4-chloro)phenyl-5-ethyl-oxazolidine

2-[N-(3,4-dichloro)phenyl]imino-3-(3-trifluoromethyl)phenyl-5-ethyl oxazolidine
2-[N-(4-fluoro)phenyl]imino-3-(3-trifluoromethyl)phenyl-5-ethyl oxazolidine
2-[N-(4-bromo)phenyl]imino-3-(3-trifluoromethyl)phenyl-5-ethyl oxazolidine

The compounds set forth above have been found to have especially good herbicidal activity against perennial and annual grasses and broadleaf weed species when applied as post-emergent herbicides. They are also effective as pre-emergence herbicides.

Preferred compounds are those wherein X is attached at the 3-position and n is 1, and Y is attached at the 3 or 4-position and n is also 1.

The foregoing, and other compounds, of the general formula set forth above (I.) can be prepared in accordance with the process of the invention which comprises: (a) reacting a urea alcohol of the formula

(1)

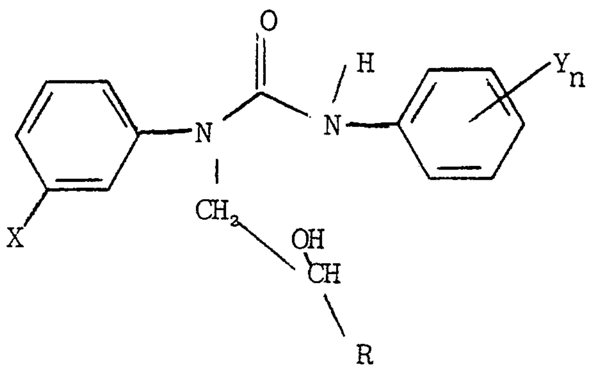

wherein x, Y, R and n are as defined above; with a dehydrating agent to form an intermediate salt compound of the formula

(2)

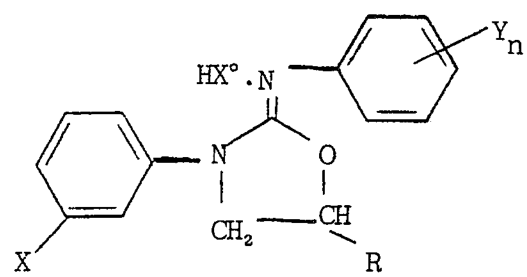

wherein x, Y, n and R are as defined above and X° is halogen or other salt forming anion and (b) reacting said salt compound with a base to form a compound of the formula

(3)

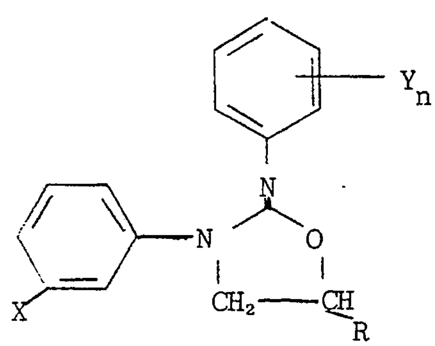

wherein X, Y, n and R are as previously defined.

The reaction sequence of the process of this invention can be represented as follows.

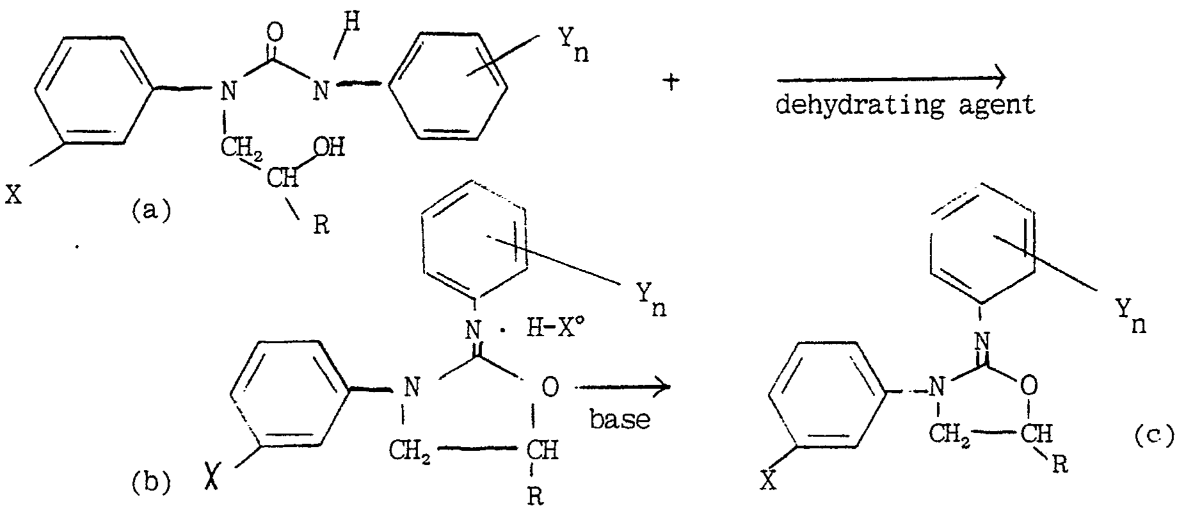


(a) (b) (c)

The urea alcohol used as the starting material in the process of the invention can be made by reacting an anilino alcohol of the type described in commonly assigned US-A- 4,723,986 with an isocyanate, wherein the reaction is conducted at room temperature and for a period of time of about one hour. The anilino alcohol used to produce the starting urea alcohol compound of the process of this invention can in turn be made by reacting a suitable aniline with an expoxide in a manner such as is also described in US-A-4,723,986.

The dehydrating agent used to dehydrate the urea alcohol starting compound of this invention can be any of the conventionally known dehydrating agents, such as thionyl chloride, phosgene, phosphorus oxychloride, phosphorus pentoxide and the like. The preferred dehydrating agent for use in the process of the invention is thionyl chloride.

The dehydrating agent is preferably used in excess when used for dehydration of the urea alcohol.

The base which is used in the second step (b) of the process of the invention serves to neutralize the intermediate salt form of the compound produced as a consequence of the dehydrating step.

The base can be any of a number of commonly known bases, including pyridine, triethylamine, sodium bicarbonate, sodium hydroxide, potassium hydroxide and the like.

The preferred base for use in the process of the invention is sodium hydroxide, although the choice of base is basically an economic factor.

The process of the invention is preferably carried out at atmospheric pressure, and at ambient temperature, which will vary depending upon the particular starting compounds used. Excessive temperature results in undesirable by-products being formed.

The time of the reaction will also vary according to the starting compounds and the temperatures used.

The intermediate salt compound (b) can be isolated and has also been found to have herbicidal activity. Thus, if desired, the process can be interrupted after the dehydrating step.

The compounds of the invention can also be produced by reacting an anilino alcohol with an isocyanate in the presence of a suitable solvent and thionyl chloride, in accordance with the following sequence of steps, the preparation of 2-[N-(3-chloro-4-methyl)phenyl]imino-3-(3-trifluromethyl)phenyl-5-ethyl oxaxolidine, one of the compounds of the invention being represented.

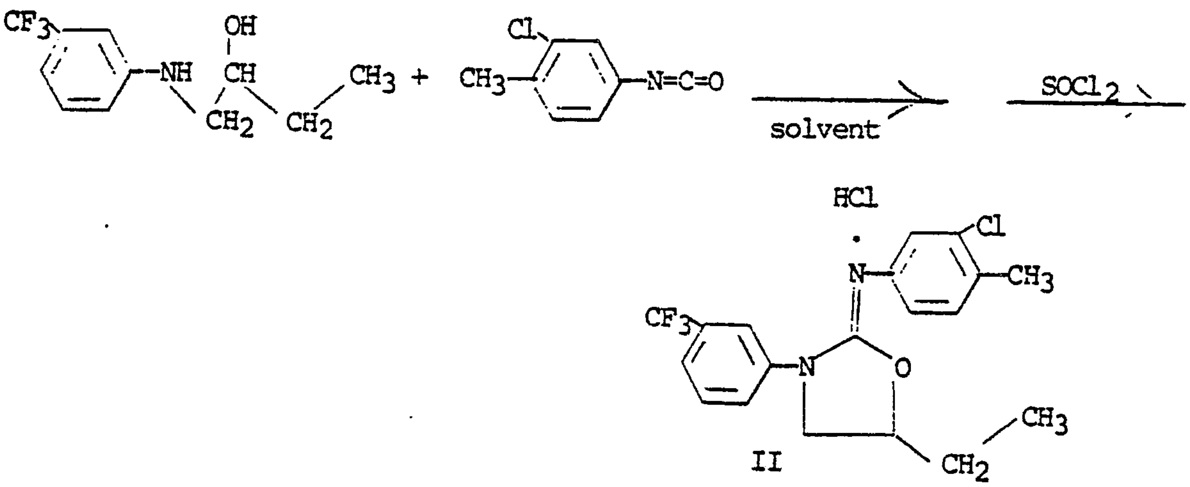


II

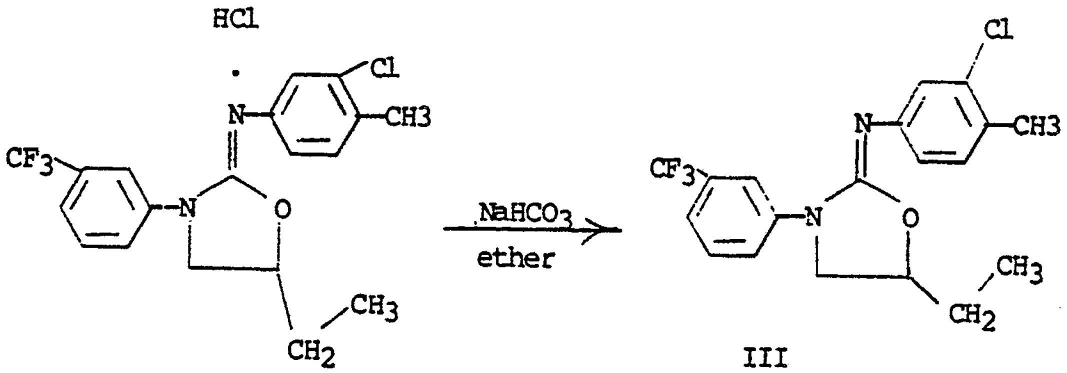


III

II

An intermediate compond produced during the course of the above reaction is the hydrochloride salt of the iminooxazolidine (II). These intermediate salt compounds also have herbicidal activity and fall within the scope of this invention.

The examples below illustrate various methods of making the compounds of the invention, using a variety of starting materials. The products were identified by suitable analytical techniques, such as NMR, IR and MS.

## EXAMPLE 1

### Preparation of 2-[N-(3-Chloro-4-methyl)phenyl]imino-3-(3-trifluoromethyl)phenyl-5-ethyl oxazolidine

A round-bottomed flask was obtained, equipped with an addition funnel and thermometer. Into this flask was combined 4.1 grams (g) (0.018 mole) of 1-(3-trifluoromethyl)anilino-2-butanol, 3.0 g (0.018 mole) of 3-chloro-4-methylphenyl isocyanate in 20 milliliters (ml) of methylene chloride. This mixture was stirred for one hour then stripped of methylene chloride at 40°C. The residual was identified as 1-(3-trifluoromethyl)-phenyl-1-(2'-hydroxy)butyl, 3-(3-chloro-4-methyl)phenyl urea.

Thereafter, 25 ml of methylene chloride and 2.4 g thionyl chloride (0.02 mole) was added to the residual material. An exothermic reaction resulted and the reaction mixture was stirred for 0.5 hour, then allowed to cool. The methylene chloride was again stripped, and the residual material triturated with an ether/pentane mixture, yielding 6.5 g of a white solid material, which was identified by suitable analytical techniques.

Thereafter, 0.42 g of this material (0.001 mole) was combined with 0.1g of a 50% solution of sodium hydroxide and 10 ml of methyl alcohol. The reaction mixture was then stirred at room temperature overnight, stripped, and subjected to a water work-up.

0.3 grams of the product was obtained, which was identified as such by suitable analytical techniques.

EXAMPLE 2

Preparation of 2-[N-(4-Chloro)phenyl]imino-3-(3-trifluoromethyl)phenyl-5-ethyl oxazolidine hydrochloride

4.5 grams of 1-(3-trifluoromethyl)anilino-2-butanol (0.0195 mole) was combined with 3 g of 4-chlorophenyl isocyanate (0.0195 mole) in 15 ml of toluene. The reaction mixture was then stirred and solids began to precipitate after about 1 hour. Thereafter, 10 additional ml of toluene were added and 2.6 g thionyl chloride (0.22 mole). The solids dissolved and the reaction mixture was stirred at room temperature overnight. IR analysis indicated that an HCl salt was present, and therefore 25 ml of pentane was added. A dark layer separated, and the mixture was then stirred at room temperature over the weekend, at which time solids precipitated. The solids were filtered, yielding 5.9 g of material, which was identified by suitable analytical techniques.

EXAMPLE 3

Preparation of 2-[N-(4-Chloro)phenyl]imino-3-(3-trifluoromethyl)phenyl-5-ethyl oxazolidine hydrochloride

The same procedure as in the previous example was repeated, except that methylene chloride was used instead of toluene as a solvent. After the thionyl chloride addition, an exotherm resulted with gas evolution, and the reaction mixture was stirred for one hour at room temperature. It was then stripped, and triturated in ether, yielding 6.8 g of material, which was identified by suitable analytical techniques.

EXAMPLE 4

Preparation of 2-[N-(4-Chloro)phenyl]imino-3-(3-trifluoromethyl)phenyl-5-ethyl oxazolidine

1.5 g (0.01 mole) of 4-chlorophenyl isocyanate was combined with 2.25 g (0.01 mole) of 1-(3-trifluoromethyl)anilino-2-butanol in 10 ml of methylene chloride. The reaction mixture was stirred for 2 hours and at the end of that time, 1.4 g (0.01 mole) of $P_2O_5$ was added all in one portion. An exotherm resulted, and the the reaction mixture was stirred for 2 hours, then water , ether, and 100 ml of one molar NAOH was added. The phases were separated, and the aqueous phase was washed with ether, with brine, dried and stripped, yielding 3.1 g of material identified as the product compound by suitable analytical techniques.

EXAMPLE 5

Preparation of 2-[N-(4-Fluoro)phenyl]imino-3-(3-trifluoromethyl)phenyl-5-ethyl oxazolidine

1.4 g (0.01 mole) of 4-fluorophenyl isocyanate was combined with 2.35 g (0.01 mole) of 1-(3-trifluoromethyl)anilino-2-butanol in 10 ml of toluene. The reaction mixture was stirred for 1 hour and at the end of that time, 1.8 g (0.011 mole) of $POCl_3$ in 5 ml methylene chloride was added all in one portion. The reaction mixture was stirred for 1 hour, then water, ether, and 100 ml of one molar NaOH was added. The phases were separated, and the aqueous phase was washed with ether, with brine, dried and stripped, yielding 3.5 g of material identified as the product compound by suitable analytical techniques.

EXAMPLE 6

Preparation of 2-[N-(4-cyano)phenyl]imino-3-(3-trifluoromethyl)phenyl-5-ethyl-oxazolidine hydrochloride salt

A round-bottom flask, fitted with a thermometer and addition funnel was charged with 2.3 g (0.01 mole)

of 1-(m-trifluoromethyl)anilino-2-butanol, 1.45 g (0.01 mole) of (4-cyano)phenyl isocyanate and 20 ml of methylene chloride. The reaction mixture was further processed as in the previous example, yielding 3.2 g of a white solid material, which was identified as the hydrochloride salt of 2-[N-(4-cyano)phenyl]imino-3-(3-trifluoromethyl)phenyl-5-oxazolidine.

A number of other compounds were prepared in accordance with the same general techniques set forth in the previous examples and are set forth in Table 1 below.

TABLE I

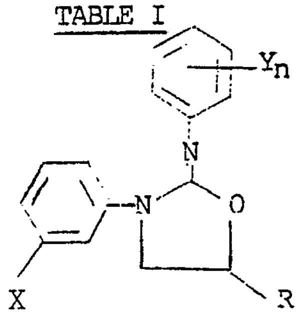

| Cmpd. No. | X | Y | R | $n_D^{30}$ or m.p. °C |
|---|---|---|---|---|
| 1 | 3-$CF_3$ | 3-$CF_3$ | $C_2H_5$ | 1.5645 |
| 2 | 3-$CF_3$ | 4-Cl | $C_2H_5$ | 1.5680 |
| 3 | 3-cyano | 4-Cl | $C_2H_5$ | 1.6145 |
| 4 | 3-cyano | 3-$CF_3$ | $C_2H_5$ | 1.5808 |
| (5 | 4-Cl | 4-Cl | $C_2H_5$ | 1.6300) |
| 6 | 3-$CF_3$ | 4-Cl | $C_2H_5$ | 79-85 |
| 7 | 3-$CF_3S$ | 4-F | $C_2H_5$ | 53-56 |
| 8 | 3-$CF_3$ | 3,4-Cl | $C_2H_5$ | 1.5675 |
| 9 | 3-$CF_3$ | 4-F | $C_2H_5$ | 1.5410 |
| 10 | 3-$CF_3$ | 4-Br | $C_2H_5$ | 1.5576 |
| 11 | 3-$CF_3$ | 4-CN | $C_2H_5$ | 189-196 |
| 12 | 3-$CF_3$ | 4-$CH_3$ | $C_2H_5$ | 1.5640 |
| 13 | 3-$CF_3$ | 4-Br | H | 1.6145 |
| 14 | 3-$CF_3$ | 3-Cl, 4-F | $C_2H_5$ | 55-60 |
| 15 | 3-$CF_3$ | 4-$OCH_3$ | $C_2H_5$ | amber oil |
| 16 | 3-$CF_3$ | 3-Cl, 4-$CH_3$ | $C_2H_5$ | amber oil |
| 17 | 3-$CF_3$ | 3-Cl | $C_2H_5$ | amber oil |
| 18 | 3-$CF_3$ | 4-$CF_3$ | $C_2H_5$ | amber oil |
| 19 | 3-$CF_3$ | 4-ethoxycarbonyl | $C_2H_5$ | amber oil |
| 20 | 3-$CF_3$ | 3-$CF_3$, 4-F | $C_2H_5$ | 95-97 |
| 21 | 3-$CF_3$ | 4-$CH_3S$ | $C_2H_5$ | 1.5985 |
| 22 | 3-$CF_3$ | 4-$SOCH_3$ | $C_2H_5$ | 1.5885 |
| 23 | 3-$CF_3$ | 2-F | $C_2H_5$ | 1.5690 |
| 24 | 3-$CF_3$ | 3-$CF_3$, 4-Cl | $C_2H_5$ | 64-66 |
| 25 | 3-$CF_3$ | 3-$SCH_3$ | $C_2H_5$ | 1.5935 |
| 26 | 3-$CF_3$ | 2,4-F | $C_2H_5$ | 46-49 |
| 27 | 3-$CF_3$ | 3-F | $C_2H_5$ | 1.5704 |
| 28 | 3-$CF_3$ | 4-$COCH_3$ | $C_2H_5$ | 77-82 |
| 29 | 3-$CF_3$ | hydrogen | $C_2H_5$ | 1.5803 |
| 30 | 3-$CF_3$ | 4-$CF_3O$ | $C_2H_5$ | 49-50 |

A series of intermediate salt compounds were also prepared in accordance with the general process as disclosed in Examples 3,5 and 6 above. These compounds are set forth in Table II below.

TABLE 2

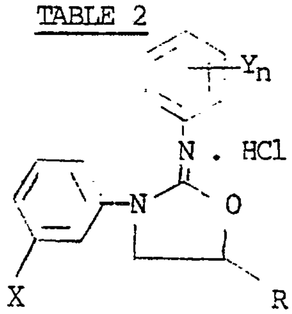

| Cmpd. No. | X | Y | R | $n_D^{30}$ or m.p. °C |
|---|---|---|---|---|
| 31 | 3-$CF_3$ | 3-Cl, 4-$CH_3$ | $C_2H_5$ | 109-112 |
| 32 | 3-$CF_3$ | 4-Cl | $C_2H_5$ | 125-127 |
| 33 | 3-$CF_3$ | 4-Fl | $C_2H_5$ | 139-141 |
| 34 | 3-$CF_3$ | 4-CN | $C_2H_5$ | 120-124 |
| 35 | 3-$CF_3$ | 3-Cl, 4-Fl | $C_2H_5$ | 138-140 |
| 36 | 3-$CF_3$ | 4-$OCH_3$ | $C_2H_5$ | 135-138 |
| 37 | 3-$CF_3$ | 3-Cl | $C_2H_5$ | 118-120 |
| 38 | 3-$CF_3$ | 4-$CF_3$ | $C_2H_5$ | 124-127 |
| 39 | 3-$CF_3$ | 4-$COOCH_2CH_3$ | $C_2H_5$ | 124-125 |
| 40 | 3-$CF_3$ | 4-$SCH_3$ | $C_2H_5$ | 128-130 |
| 41 | 3-$CF_3S$ | 4-Fl | $C_2H_5$ | 125-127 |
| 42 | 3-$CF_3$ | 4-$OCH_3$ | $C_2H_5$ | 125-127 |
| 43 | 3-$CF_3$ | 2-Fl | $C_2H_5$ | 124-125 |
| 44 | 3-$CF_3$ | 3-$CF_3$, 4-Cl | $C_2H_5$ | 116-119 |
| 45 | 3-$CF_3$ | 3-$CH_3$ | $C_2H_5$ | 100-105 |
| 46 | 3-$CF_3$ | 2,4-Fl | $C_2H_5$ | 114-119 |
| 47 | 3-$CF_3$ | 3-Fl | $C_2H_5$ | 117-119 |
| 48 | 3-$CF_3$ | 4-$COCH_3$ | $C_2H_5$ | 97-100 |
| 49 | 3-$CF_3$ | hydrogen | $C_2H_5$ | 139-140 |

The herbicidal activity of representative ones of the compounds of the invention are exhibited by means of tests in accordance with the following procedures.

EXAMPLE 7

Herbicidal Activity Tests

This example offers additional herbicidal activity test data to show the effectiveness of the compounds of the invention against various weed species. The effect is observed by comparing the extent of weed control in test flats treated with the compounds against that occurring in similar control flats. The soil used in these tests was a sandy loam soil from the Livermore, California area.

Also added to the soil was 17-17-17 fertilizer ($N-P_2O_5-K_2O$ on a weight basis), amounting to 50 ppm by weight with respect to the soil and 100 ppm Captan, a soil fungicide.

The treated soil was then placed in flats which were 3 inches deep, 6 inches wide, and 10 inches long (7.62 x 15.24 x 25.4 cms). The soil was tamped and leveled width of the flat. The test weeds were as follows:

| COMMON NAME | | SCIENTIFIC NAME | ABR |
|---|---|---|---|
| Broadleaf Weeds: | | | |
| A. | annual morningglory | *Ipomoea purpurea* | PHBPU |
| B. | velvetleaf | *Abutilon theophrasti* | ABUTY |
| C. | mustard | *Brassica kaber* | SINAR |
| D. | curly dock | *Rumex crispus* | RUMCR |
| Grasses: | | | |
| D. | yellow nutsedge | *Cyperus exculentus* | CYPES |
| E. | foxtail | *Setaria sp.* | SETVI |
| F. | watergrass | *Echinochloa crusgalli* | ECHOG |
| G. | wild oat | *Avena fatua* | AVEFA |

Sufficient seeds were planted to produce several seedlings per inch in each row. The flats were then placed in a greenhouse maintained at 70 to 85° F (21 to 30° C) and watered daily by sprinkler.

In the case of pre-emergent testing (PES) the herbicide was applied to the soil after planting of the seeds, at a rate equivalent to the indicated amounts in the Table.

In post-emergent testing (POS) chemical application is made by spraying 12 days after planting. The spray solution is prepared by dissolving 60 mg of herbicide compound in 20 ml of acetone containing 1% Tween® 20 (polyoxysorbitan monolaurate), then adding 20 ml of water to the resulting solution. The solution is sprayed at 80 US gallon/acre(122.6 1/Ha), resulting in a 4 lb/acre(4.48 KG/Ha) rate of chemical application.

In both instances, either pre- or post-emergent testing, approximately 12-14 days after treatment, the degree of weed control was estimated and recorded as percentage control compared to the growth of the same species in an untreated check flat of the same age. The rating scale ranges from 0 to 100%, where 0 equals no effect with plant growth equal to the untreated control, and 100 equals complete kill.

The results are listed in the Table below.

TABLE 3

GREENHOUSE HERBICIDE TEST RESULTS —

| Test Compound No. | Application Rate (lb/A) | Application Method | Percent Injury SETVI | ECHCG | AVEFA | PHBPU | ABUTH | SINAR | RUMCR | CYPES |
|---|---|---|---|---|---|---|---|---|---|---|
| 1 | 4.00 | PES | 100 | 75 | 25 | 10 | 25 | 95 | 95 | 0 |
| | 4.00 | POS | 90 | 25 | 25 | 85 | 90 | 70 | 85 | N |
| 2 | 4.00 | PES | 100 | 100 | 35 | 85 | 70 | 100 | 100 | 0 |
| | 4.00 | POS | 95 | 75 | 85 | 75 | 85 | 85 | 100 | 20 |
| 3 | 4.00 | PES | 95 | 75 | 0 | 90 | 50 | 100 | 95 | 0 |
| | 4.00 | POS | 10 | 10 | 0 | 20 | 35 | 35 | 50 | 0 |
| 4 | 4.00 | PES | 100 | 70 | 10 | 10 | 10 | 10 | 95 | 0 |
| | 4.00 | POS | 20 | 25 | 0 | 80 | 80 | 90 | 60 | 0 |
| 5 | 4.00 | PES | 85 | 0 | 0 | 10 | 0 | 20 | 90 | 0 |
| | 4.00 | POS | 0 | 0 | 0 | 15 | 20 | 60 | 40 | 0 |
| 6 | 4.00 | PES | 0 | 0 | 0 | 0 | 0 | 0 | 0 | 0 |
| | 4.00 | POS | 0 | 0 | 0 | 0 | 0 | 50 | 0 | 0 |
| 7 | 4.00 | PES | 100 | 70 | 40 | 60 | 35 | 100 | 100 | 0 |
| | 4.00 | POS | 85 | 70 | 25 | 35 | 40 | 90 | 100 | 10 |
| 8 | 4.00 | PES | 100 | 100 | 80 | 70 | 90 | 100 | 100 | 0 |
| | 4.00 | POS | 90 | 75 | 40 | 70 | 65 | 85 | 100 | 0 |
| 9 | 4.00 | PES | 100 | 85 | 35 | 70 | 50 | 100 | 100 | 0 |
| | 4.00 | POS | 50 | 70 | 40 | 70 | 70 | 75 | 95 | 10 |
| 10 | 4.00 | PES | 90 | 75 | 35 | 75 | 60 | 75 | 30 | 0 |
| | 4.00 | POS | 90 | 40 | 30 | 20 | 20 | 35 | 25 | N |
| 11 | 4.00 | PES | 95 | 80 | 40 | 50 | 30 | N | 65 | 0 |
| | 4.00 | POS | 70 | 60 | 35 | 85 | 80 | 85 | 80 | 0 |
| 12 | 4.00 | PES | 80 | 75 | 25 | 25 | 20 | 35 | 80 | 0 |
| | 4.00 | POS | 30 | 30 | 30 | 50 | 35 | 50 | 75 | 10 |
| 13 | 4.00 | PES | 100 | 100 | 80 | 90 | 85 | 95 | N | 0 |
| | 4.00 | POS | 100 | 90 | 80 | 90 | 100 | 100 | N | 0 |
| 14 | 4.00 | PES | 100 | 100 | 80 | 100 | 95 | 100 | N | 0 |
| | 4.00 | POS | 95 | 80 | 80 | 60 | 90 | 80 | N | 5 |
| 15 | 4.00 | PES | 100 | 100 | 75 | 100 | 60 | 100 | N | 0 |
| | 4.00 | POS | 50 | 70 | 60 | 60 | 80 | 80 | N | 0 |
| 16 | 4.00 | PES | 100 | 80 | 80 | 80 | 80 | 100 | N | 0 |
| | 4.00 | POS | 50 | 70 | 50 | 60 | 90 | 80 | N | 0 |

TABLE 3
(continued)

| Test Compound No. | Application Rate (lb/A) | Method | Percent Injury SETVI | ECHCG | AVEFA | PHBPU | ABUTH | SINAR | RUMCR | CYPES |
|---|---|---|---|---|---|---|---|---|---|---|
| 17 | 4.00 | PES | 100 | 100 | 80 | 100 | 95 | 100 | N | 0 |
| | 4.00 | POS | 80 | 70 | 80 | 60 | 80 | 80 | N | 0 |
| 18 | 4.00 | PES | 100 | 100 | 90 | 100 | 100 | 100 | N | 0 |
| | 4.00 | POS | 60 | 80 | 80 | 50 | 90 | 80 | N | 0 |
| 19 | 4.00 | PES | 95 | 80 | 70 | 20 | 80 | 100 | N | 0 |
| | 4.00 | POS | 10 | 30 | 20 | 60 | 80 | 80 | N | 0 |
| 20 | 4.00 | PES | 100 | 95 | 80 | 100 | 100 | 100 | N | 0 |
| | 4.00 | POS | 90 | 100 | 90 | 90 | 95 | 95 | N | 0 |
| 21 | 4.00 | PES | 100 | 85 | 50 | 100 | 95 | 100 | N | 0 |
| | 4.00 | POS | 100 | 85 | 80 | 100 | 100 | 95 | N | 5 |
| 22 | 4.00 | PES | 100 | 90 | 50 | 90 | 100 | 100 | N | 0 |
| | 4.00 | POS | 90 | 85 | 60 | 40 | 90 | 90 | N | 10 |
| 23 | 4.00 | PES | 100 | 85 | 70 | 40 | 50 | 100 | N | 0 |
| | 4.00 | POS | 85 | 50 | 40 | 80 | 80 | 80 | N | 0 |
| 24 | 4.00 | PES | 100 | 85 | 70 | 90 | 95 | 100 | N | 0 |
| | 4.00 | POS | 80 | 50 | 40 | 80 | 90 | 80 | N | 0 |
| 25 | 4.00 | PES | 100 | 85 | 70 | 100 | 90 | 100 | N | 0 |
| | 4.00 | POS | 80 | 60 | 50 | 100 | 90 | 90 | N | 30 |
| 26 | 4.00 | PES | 100 | 100 | 80 | 90 | 90 | 100 | N | 0 |
| | 4.00 | POS | 85 | 80 | 60 | 80 | 80 | 90 | N | 0 |
| 27 | 4.00 | PES | 100 | 100 | 85 | 100 | 95 | 100 | N | 0 |
| | 4.00 | POS | 95 | 90 | 80 | 100 | 100 | 100 | N | 0 |
| 28 | 4.00 | PES | 100 | 40 | 20 | 40 | 50 | 100 | N | 0 |
| | 4.00 | POS | 30 | 20 | 10 | 60 | 80 | 80 | N | 0 |
| 29 | 4.00 | PES | 100 | 100 | 75 | 100 | 95 | 100 | N | 0 |
| | 4.00 | POS | 95 | 85 | 60 | 80 | 90 | 90 | N | 0 |
| 30 | 4.00 | PES | 100 | 90 | 80 | 80 | 100 | 100 | N | N |
| | 4.00 | POS | 85 | 60 | 50 | 80 | 80 | 80 | N | 10 |
| 31 | 4.00 | PES | 100 | 80 | 70 | 80 | 60 | 100 | N | 0 |
| | 4.00 | POS | 20 | 50 | 40 | 50 | 90 | 90 | N | 0 |
| 32 | 4.00 | PES | 100 | 100 | 80 | 100 | 90 | 100 | N | 0 |
| | 4.00 | POS | 90 | 80 | 80 | 60 | 80 | 80 | N | 0 |
| 33 | 4.00 | PES | 100 | 100 | 90 | 100 | 100 | 100 | N | 0 |
| | 4.00 | POS | 90 | 85 | 90 | 85 | 90 | 80 | N | 0 |

TABLE 3
(continued)

| Test Compound No. | Application Rate (lb/A) | Method | Percent Injury SETVI | ECHCG | AVEFA | PHBPU | ABUTH | SINAR | RUMCR | CYPES |
|---|---|---|---|---|---|---|---|---|---|---|
| 34 | 4.00 | PES | 100 | 100 | 80 | 100 | 100 | 100 | N | 0 |
| | 4.00 | POS | 80 | 60 | 80 | 50 | 90 | 80 | N | 0 |
| 35 | 4.00 | PES | 100 | 100 | 80 | 95 | 90 | 100 | N | 0 |
| | 4.00 | POS | 95 | 90 | 80 | 0 | 30 | 80 | N | 0 |
| 36 | 4.00 | PES | 100 | 100 | 80 | 80 | 85 | 100 | N | 0 |
| | 4.00 | POS | 85 | 80 | 70 | 80 | 90 | 80 | N | 0 |
| 37 | 4.00 | PES | 100 | 100 | 80 | 40 | 80 | 100 | N | 0 |
| | 4.00 | POS | 85 | 80 | 70 | 60 | 90 | 80 | N | 0 |
| 38 | 4.00 | PES | 100 | 100 | 85 | 90 | 95 | 100 | N | 0 |
| | 4.00 | POS | 50 | 80 | 80 | 50 | 90 | 80 | N | 0 |
| 39 | 4.00 | PES | 100 | 50 | 30 | 20 | 20 | 80 | N | 0 |
| | 4.00 | POS | 10 | 30 | 5 | 60 | 90 | 80 | N | 0 |
| 40 | 4.00 | PES | 100 | 85 | 50 | 85 | 95 | 100 | N | 0 |
| | 4.00 | POS | N | N | N | N | N | N | N | N |
| 41 | 4.00 | PES | 100 | 90 | 80 | 90 | 90 | 100 | N | 0 |
| | 4.00 | POS | 100 | 80 | 80 | 90 | 100 | 100 | N | 0 |
| 42 | 4.00 | PES | 100 | 50 | 95 | 100 | 100 | 80 | N | N |
| | 4.00 | POS | 80 | 30 | 70 | 80 | 80 | 80 | N | 20 |
| 43 | 4.00 | PES | 100 | 85 | 70 | 50 | 50 | 95 | N | 0 |
| | 4.00 | POS | 90 | 50 | 30 | 80 | 80 | 80 | N | 0 |
| 44 | 4.00 | PES | 100 | 85 | 70 | 60 | 50 | 100 | N | 0 |
| | 4.00 | POS | 95 | 50 | 30 | 80 | 80 | 80 | N | 0 |
| 45 | 4.00 | PES | 100 | 85 | 60 | 100 | 95 | 100 | N | 0 |
| | 4.00 | POS | 80 | 60 | 60 | 100 | 100 | 100 | N | 40 |
| 46 | 4.00 | PES | 100 | 100 | 80 | 90 | 90 | 100 | N | 0 |
| | 4.00 | POS | 95 | 80 | 60 | 60 | 85 | 85 | N | 0 |
| 47 | 4.00 | PES | 100 | 100 | 85 | 90 | 90 | 100 | N | 0 |
| | 4.00 | POS | 100 | 80 | 60 | 90 | 85 | 80 | N | 0 |
| 48 | 4.00 | PES | 100 | 20 | 20 | 40 | 40 | 100 | N | 0 |
| | 4.00 | POS | 5 | 10 | 5 | 10 | 50 | 80 | N | 0 |
| 49 | 4.00 | PES | 100 | 100 | 85 | 100 | 90 | 100 | N | 0 |
| | 4.00 | POS | 100 | 85 | 80 | 100 | 100 | 90 | N | 0 |

N = Not tested. (4 lb/A = 4.48 Kg/Ha)

## METHODS OF APPLICATION

The herbicidal compositions of the present invention are useful in controlling the growth of undesirable vegetation by pre-emergence or post-emergence application to the locus where control is desired, including pre-plant and post-plant soil incorporation as well as the surface application. The compositions are generally embodied in formulations suitable for convenient application. Typical formulations contain additional ingredients or diluent carriers which are either inert or active. Examples of such ingredients or carriers are water, organic solvents, dust carriers, granular carriers, surfact active agents, oil and water, water-oil emulsions, wetting agents, dispersing agents, and emulsifying agents. The herbicidal formulations generally take the form of dusts, emulsifiable concentrates, granules and pellets, or microcapsules.

### A. DUSTS

Dusts are dense powder compositions which are intended for application in dry form. Dusts are characterized by their free-flowing and rapid settling properties so that they are not readily windborne to areas where their presence is not desired. They contain primarily an active material and a dense, free-flowing, solid carrier.

Their performance is sometimes aided by the inclusion of a wetting agent, and convenience in manufacture frequently demands the inclusion of an inert, absorptive grinding aid. For the dust compositions of this invention, the inert carrier may be either of vegetable or mineral origin, the wetting agent is preferably anionic or nonionic, and suitable absorptive grinding aids are of mineral origin.

Suitable classes of inert solid carriers for use in the dust compositions are those organic or inorganic powders which possess high bulk density and are very free-flowing. They are also characterized by low surface area and poor liquid absorptivity. Suitable grinding aids are natural clays, diatomaceous earths, and synthetic mineral fillers drived from silica or silicate. Among ionic and nonionic wetting agents, the most suitable are the members of the group known to the art as wetting agents and emulsifiers. Although solid agents are preferred because of ease of incorporation, some liquid nonionic agents are also suitable in the dust formulations.

Preferred dust carriers are micaceous talcs, pyrophyllite, dense kaolin clays, tobacco dust and ground calcium phosphate rock.

Preferred grinding aids are attapulgite clay, diatomaceous silica, synthetic fine silica and synthetic calcium and magnesium silicates.

Most preferred wetting agents are alkylbenzene and alkyl-naphthalene sulfonates, sulfated fatty alcohols, amines or acid amides, long chain acid esters of sodium isothionate, esters of sodium sulfosuccinate, sulfated or sulfonated fatty acid esters, petroleum sulfonates, sulfonated vegetable oils, and ditertiary acetylenic glycols. Preferred dispersants are methyl cellulose, polyvinyl alcohol, lignin sulfonates, polymeric alkylnaphthalene sulfonates, sodium naphthalenesulfonate, polymethylene bisnaphthalenesulfonate, and sodium-N-methyl-N-(long chain acid) taurates.

The inert solid carriers in the dusts of this invention are usually present in concentrations of from about 30 to 90 weight percent of the total composition. The grinding aid will usually constitute 5 to 50 weight percent of the compositions, and the wetting agent will constitute from about 0 to 1.0 weight percent of the composition. Dust compositions can also contain other surfactants such as dispersing agents in concentrations of up to about 0.5 weight percent, and minor amounts of anticaking and antistatic agents. The particle size of the carrier is usually in the range of 30 to 50 microns ($\mu$m).

### B. EMULSIFIABLE CONCENTRATES

Emulsifiable concentrates are usually solutions of the active materials in nonwater-miscible solvents together with an emulsifying agent. Prior to use, the concentrate is diluted with water to form a suspended emulsion of solvent droplets.

Typical solvents for use in emulsifiable concentrates include weed oils, chlorinated hydrocarbons, and nonwater-miscible ethers, esters, and ketones.

Typical emulsifying agents are anionic or nonionic surfactants, or mixtures of the two. Examples include

long-chain alkyl or mercaptan polyethoxy alcohols, alkylaryl polyethoxy alcohols, sorbitan fatty acid esters, polyoxyethylene ethers with sorbitan fatty acid esters, polyoxyethylene glycol esters with fatty or rosin acids, fatty alkylol amide condensates, calcium and amine salts of fatty alcohol sulfates, oil soluble petroleum sulfonates, or preferably mixtures of these emulsifying agents. Such emulsifying agents will comprise from about 1 to 10 weight percent of the total composition.

Thus, emulsifiable concentrates of the present invention will consist of from about 15 to about 50 weight percent active material, about 40 to 82 weight percent solvent, and about 1 to 10 weight percent emulsifier. Other additives such as spreading agents and stickers can also be included.

C. GRANULES AND PELLETS

Granules and pellets are physically stable, particulate compositions containing the active ingredients adhering to or distributed through a basic matrix of a coherent, inert carrier with microscopic dimensions. A typical particle is about 1 to 2 millimeters in diameter. Surfactants are often present to aid in leaching of the active ingredient from the granule or pellet.

The carrier is preferably of mineral origin, and generally falls within one of two types. The first are porous, absorptive, preformed granules, such as preformed and screened granulat attapulgite or heat expanded, granular, screened vermiculite. On either of these, a solution of the active agent can be sprayed and will be absorbed at concentrations up to 25 weight percent of the total weight. The second, which are also suitable for pellets, are initially powered kaolin clays, hydrated attapulgite, or bentonite clays in the form of sodium, calcium, or magnesium bentonites. Water-soluble salts, such as sodium salts, may also be present to aid in the disintegration of granules or pellets in the presence of moisture. These ingredients are blended with the active components to give mixtures that are granulates or pelleted, followd by drying, to yield formulations with the active component distributed uniformly throughout the mass. Such granules and pellets can also be made with 25 to 30 weight percent active component, but more frequently a concentration of about 10 weight percent is desired for optimum distribution. The granular compositions of this invention are most useful in a size range of 1530 mesh.

The surfactant is generally a common wetting agent of anionic or nonionic character. The most suitable wetting agents depend upon the type of granule used. When preformed granules are sprayed with active material in liquid form the most suitable wetting agents are nonionic, liquid wetters miscible with the solvent. These are compounds most generally known in the art as emulsifiers, and comprise alkylaryl polyether alcohols, alkyl polyether alcohols, polyoxyethylene sorbitan fatty acid esters, polyethylene glycol esters with fatty or rosin acids, f atty alkylol amide condensates, oil solution petroleum or vegetable oil sulfonates, or mixtures of these. Such agents will usually comprise up to about 5 weight percent of the total composition.

When the active ingredient is first mixed with a powdered carrier and subsequently granulated, or pelleted, liquid nonionic wetters can still be used, but it is usually preferable to incorporate at the mixing stage one of the solid, powdered anionic wetting agents such as those previously listed for the wettable powders. Such agents will comprise from about 0 to 2 weight percent of the total composition.

Thus, the preferred granular or pelleted formulations of this invention comprise about 5 to 30 percent by weight active material, about 0 to 5 weight percent wetting agent, and about 65 and 95 weight percent inert material carrier, as these terms are used herein.

D. MICROCAPSULES

Microcapsules consist of fully enclosed droplets or granules containing the active materials, in which the enclosing material is an inert porous membrane, arranged to allow escape of the enclosed materials to the surrounding medium at controlled rates over a specified period. Encapsulated droplets are typically about 1 to 50 microns ($\mu$m)in diameter.

The enclosed liquid typically constitutes about 50 to 95% of the weight of the entire capsule, and may contain a small amount of solvent in addition to the active materials.

Encapsulated granules are characterized by porous membranes sealing the openings of the granule carrier pores, trapping the liquid containing the active components inside for controlled release. A typical granule size ranges from 1 millimeter to 1 centimeter in diameter. In agricultural useage, the granule size is generally about 1 to 2 ml in diameter. Granules formed by extrusion, agglomeration, or prilling are useful in the present invention as well as materials in their naturally occurring form. Examples of such carriers are vermiculite, sintered clay granules, kaolin, attapulgite clay, sawdust, and granular carbon.

Useful encapsulating materials include natural and synthetic rubbers, cellulosic materials, styrene-butadiene copolymers, polyacrylonitriles, polyacrylates, polyesters, polyamides, polyurethanes, and starch xanthates.

E. IN GENERAL

Each of the above formulations can be prepared as a package containing the herbicide together with the other ingredients of the formulation (diluents, emulsifiers, surfactants, etc.). The formulations can also be prepared by a tank mix method, in which the ingredients are obtained separately and combined at the grower site.

In general, any conventional method of application can be used. The locus of application can be soil, seeds, seedlings, or the actual plants, as well as flooded fields. Post-emergent application is preferred. Dusts and liquid compositions can be applied by the use of powder dusters, boom and hand sprayers, and spray dusters. The compositions can also be applied from airplanes as dusts and sprays because they are effective in very low dosages. In order to modify or control the growth of germinating seeds or emerging seedlings, as a typical example, the dust and liquid compositions are applied to the soil according to conventional methods and are distributed in the soil to a depth of at least one-half inch below the soil surface. It is not necessary that the phytotoxic compositions be admixed with the soil particles. Instead, these compositions can be applied merely by spraying or sprinkling the surface of the soil. The phytotoxic compositions of this invention can also be applied by addition to irrigation water supplied to the field to be treated. This method of application permits the penetration of the compositions into the soil as the water is absorbed therein. Dust compositions, granular compositions or liquid formulations applied to the surface of the soil can be distributed below the surface of the soil by conv entional means such as discing, dragging or mixing operations.

The herbicide compositions can also be applied to the soil through irrigation systems. According to this technique, the compositions are added directly to irrigation water immediately prior to irrigation of the field. This technique is applicable in all geographical areas regardless of rainfall, since it permits supplementation of the natural rainfall at critical stages of plant growth. In a typical application, the concentration of the herbicide composition in the irrigation water will range from about 10 to 150 parts per million by weight. The irrigation water can be applied by the use of sprinkler systems, surface furrows, or flooding. Such application is most effectively done before the weeds germinate, either early in the spring prior to germination or within two days after cultivation of the field.

The amount of the present composition which constitutes a herbicidally effective amount depends upon the nature of the seeds or plants to be controlled. The rate of application of active ingredient varies from about 0.01 to about 50 pounds per acre, preferably about 0.1 to about 25 pounds per acre with the actual amount depending on the overall cost and the desired results. It will be readily apparent to one skilled in the art that compositions exhibiting lower herbicidal activity will require a high dosage than more active compounds for the same degree of contro l .

**Claims**

**1.** An iminooxazolidine characterised in that it corresponds to the following general formula:

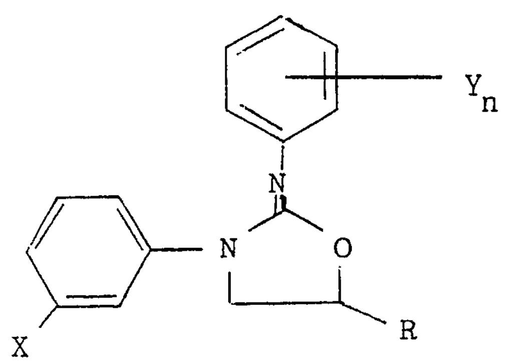

wherein

x represents cyano, halogen, alkyl, alkylthio, haloalkyl, haloalkylthio, alkylsulfenyl, alkoxy, carboalkoxy and haloalkoxy wherein the alkyl groups have from 1 to 5 carbon atoms; Y represents hydrogen, cyano, halogen, alkyl, alkylthio, haloalkyl, haloalkylthio, alkylsulfenyl, alkoxy, carboalkoxy and haloalkoxy wherein the alkyl groups have from 1 to 5 carbon atoms; n represents 1 or 2; and R represents hydrogen or a lower alkyl group having from 1 to 3 carbon atoms;
and herbicidally effective salt thereof.

2. An iminooxazolidine salt as claimed in claim 1 wherein the salt-forming moiety is HX° wherein X° represents halogen.

3. A compound as claimed in claim 1 or claim 2 wherein R represents ethyl.

4. A compound as claimed in any of claims 1 to 3 wherein X represents 3-trifluoromethyl; Y represents 4-chloro or 4-fluoro; and A represents ethyl.

5. A herbicidal composition characterised in that it comprises a compound as claimed in any of claims 1 to 4 and an agriculturally-acceptable carrier or diluent.

6. A method for controlling undesirable weed pests characterised in that it comprises applying to a locus where the control is desired an herbicidally effective amount of a compound as claimed in any of claims 1 to 4 or a composition as claimed in claim 5.

7. A process for the preparation of a compound as claimed in any of claims 1 to 4 characterised in that it comprises: (a) reacting a urea alcohol corresponding to the following general formula:

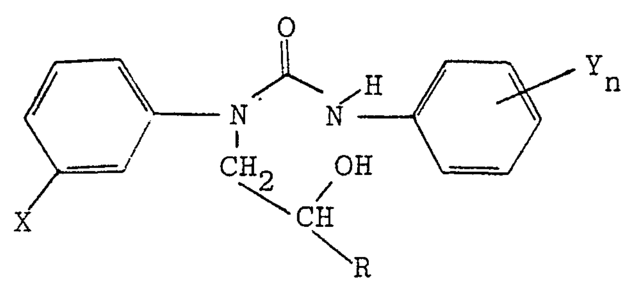

wherein X, Y, n and R are as defined above; with a dehydrating agent to form a salt compound corresponding to the following general formula:

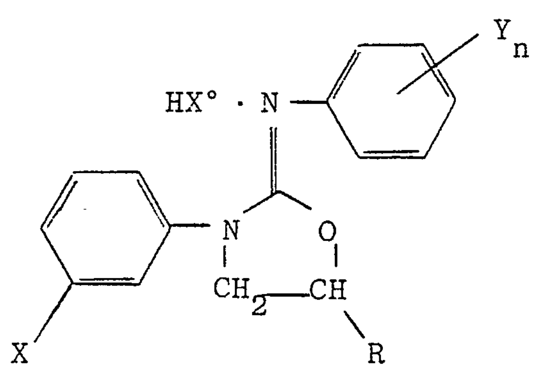

wherein X, Y, n and R are as defined above and X° represents a salt-forming anion; and; optionally, (b) reacting the salt compound with a base to form a compound corresponding to the following general formula:

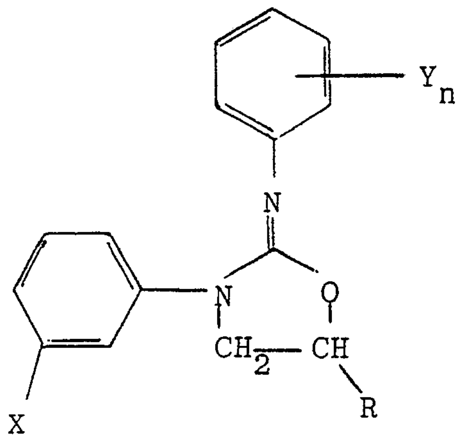

wherein n, X, Y and R are as defined above.

**8.** A process as claimed in claim 7 wherein the dehydrating agent is selected from thionyl chloride, phosgene, phosphorous oxychloride and phosphorus pentoxide.

**9.** A process as claimed in claim 7 or claim 8 wherein the base is selected from pyridine, triethylamine, sodium bicarbonate, sodium hydroxide and potassium hydroxide.

Claims for the following contracting states: ES, GR.

**1.** A herbicidal composition characterised in that it comprises an iminooxazolidine corresponding to the following general formula:

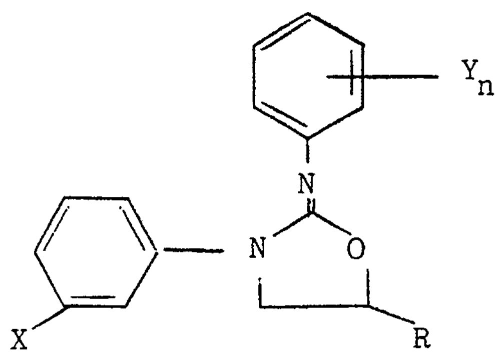

wherein

x represents cyano, halogen, acyl, alkylthio, haloalkyl, haloalkylthio, alkylsulfenyl, alkoxy, carboalkoxy and haloalkoxy wherein the alkyl groups have from 1 to 5 carbon atoms; Y represents hydrogen, cyano, halogen, alkyl, alkylthio, haloalkyl, haloalkylthio, alkylsulfenyl, alkoxy, carboalkoxy and haloalkoxy wherein the alkyl groups have from 1 to 5 carbon atoms; n represents 1 or 2; and R represents hydrogen or a lower alkyl group having from 1 to 3 carbon atoms;
or an herbicidally effective salt thereof and an agriculturally-acceptable carrier or diluent.

**2.** A composition as claimed in claim 1 wherein, in the case of an iminooxazolidine salt, the salt-forming moiety is $HX^{\circ}$ wherein $X^{\circ}$ represents halogen.

**3.** A composition as claimed in claim 1 or claim 2 wherein, in the iminooxazolidine, R represents ethyl.

**4.** A composition as claimed in any of claims 1 to 3 wherein, in the iminooxazolidine, x represents 3-trifluoromethyl; Y represents 4-chloro or 4-fluoro; and R represents ethyl.

5. A process for the production of a herbicidal composition as claimed in any of claims 1 to 4 characterised in that it comprises mixing a compound as defined in any of claims 1 to 4 with an agriculturally-acceptable carrier or diluent.

6. A method for controlling undesirable weed pests characterised in that it comprises applying to a locus where the controlis desired an herbicidally effenctive amount of a composition as claimed in any of claims 1 to 4 or a compound as defined in any of claims 1 to 4.

7. A process for the preparation of a compound as defined in any of claims 1 to 4 characterised in that it comprises: (a) reacting a urea alcohol corresponding to the following general formula:

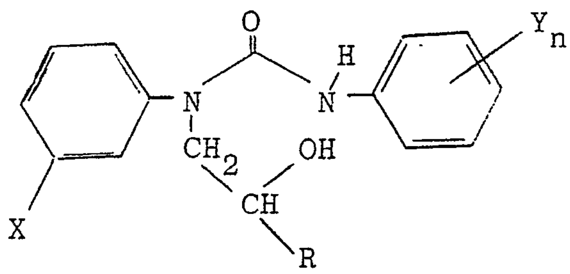

wherein X, Y, n and R are as defined above; with a dehydrating agent to form a salt compound corresponding to the following general formula:

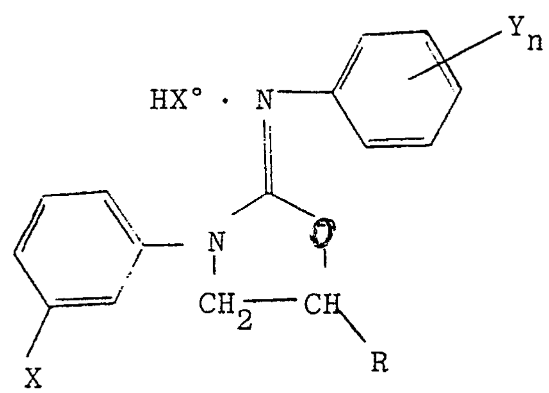

wherein X, Y, n and R are as defined above and X° represents a salt-forming anion; and, optionally, (b) reacting the salt compound with a base to form a compound corresponding to the following general formula:

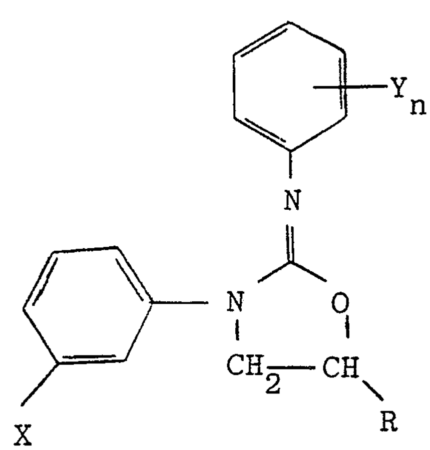

wherein n, X, Y and R are as defined above.

8. A process as claimed in claim 7 wherein the dehydrating agent is selected from thionyl chloride, phosgene, phosphorous oxychloride and phosphorus pentoxide.

9. A process as claimed in claim 7 or claim 8 wherein the base is selected from pyridine, triethylamine, sodium bicarbonate, sodium hydroxide and potassium hydroxide.

**Revendications**

1. Une iminooxazolidine, caractérisée en ce qu'elle correspond à la formule générale suivante:

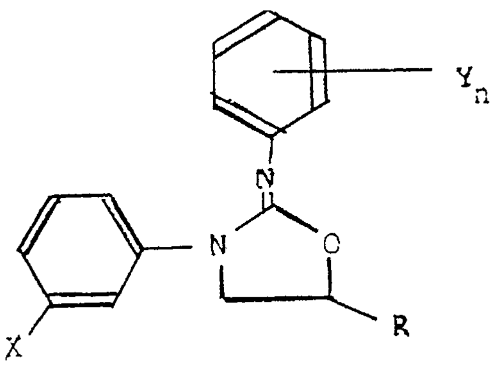

dans laquelle

X représente un groupe cyano, un atome d'halogène, un groupe alkyle, alkyithio, haloalkyle, haloalkylthio, alkylsulfényle, alcoxy, carboalcoxy et haloalcoxy, dans lesquels, les groupes alkyles ont de 1 à 5 atomes de carbone;
Y est un atome d'hydrogène, d'halogène, un groupe cyano, alkyle alkylthio, haloalkyle, haloalkylthio, alkylsulfényle, alcoxy, carboalcoxy et haloalcoxy, dans lesquels les groupes alkyles ont de 1 à 5 atomes de carbone;
n représente 1 ou 2; et
A représente un atome d'hydrogène ou un groupe alkyle inférieur ayant 1 à 3 atomes de carbone; et un sel de celle-ci efficace du point de vue herbicide.

2. Un sel d'iminooxazolidine suivant la revendication 1, caracterise en ce que la partie formant un sel est HX', où X' représente un atome d'halogène.

3. Un composé suivant la revendication 1 ou la revendication 2, caractérisé en ce que A représente un groupe ethyle.

4. Un composé suivant l'une quelconque des revendications 1 à 3, caractérisé en ce que X représente un groupe 3-trifluorométhyle; Y représente un atome 4-chloro ou 4-fluoro; et A représente un groupe éthyle.

5. Une composition herbicide, caractérisée en ce qu'elle comprend un composé suivant l'une quelconque des revendications 1 à 4 et un support ou un diluant acceptable du point de vue agricole.

6. Une méthode pour contrôler des mauvaises herbes indésirables, caractérisée en ce qu'elle comprend l'application sur un site devant être contrôlé d'une quantité efficace du point de vue herbicide d'un composé suivant l'une quelconque des revendications 1 a ou d'une composition suivant la revendication 5.

7. Un procédé pour la préparation d'un compose suivant l'une quelconque des revendications 1 a 4, caractérisé en ce qu'il comprend:
(a) la réaction d'un urée alcool correspondant à la formule générale suivante:

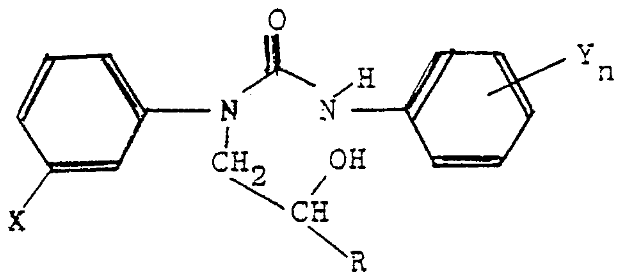

dans laquelle X,Y, n et A sont tels que définis plus haut,avec un agent déshydratant pour former un sel correspondant à la formule générale suivante:

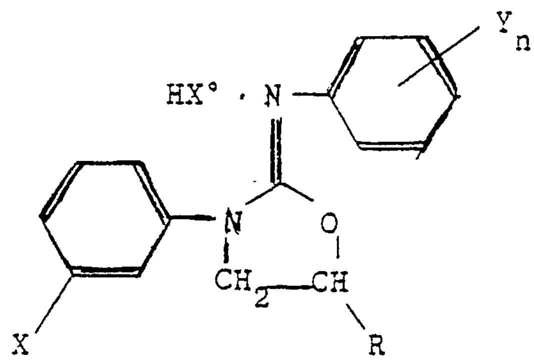

dans laquelle X,Y, n et A sont tels que définis plus haut et X' représente un anion formant un sel; et, éventuellement,

(b) la réaction du sel avec une base pour former un composé correspondant à la formule générale:

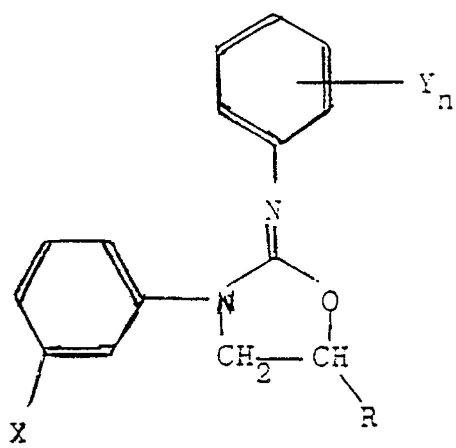

dans laquelle n,X,Y et A sont tels que définis plus haut.

**8.** Un procédé suivant la revendication 7, caractérisé en ce que l'agent déshydratant est choisi parmi le chlorure de thionyle le phosgène, l'oxychlorure de phosphore et le pentoxyde de phosphore.

**9.** Un procédé suivant la revendication 1 ou la revendication 8,caractérisé en ce que la base est choisie parmi la pyridine, la triéthylamine, le carbonate acide de sodium l'hydroxyde de sodium et l'hydroxyde de potassium.

Revendications pour les Etats contractants suivants: ES, GR.

1. Une composition herbicide, caractérisée en ce qu'elle comprend une iminooxazolidine correspondant à la formule générale suivante:

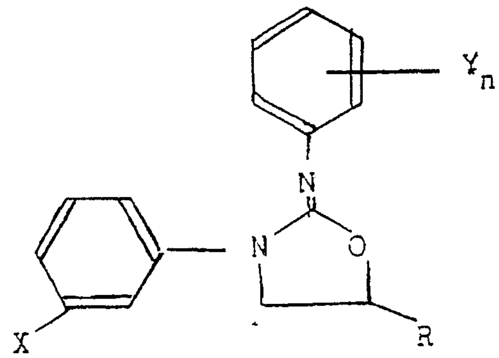

dans laquelle

- X représente un groupe cyano, un atome d'halogéne, un groupe alkyle, alkylthio, haloalkyle, haloalkylthio, alkylsulfényle, alcoxy, carboalcoxy et haloalcoxy, dans lesquels les groupes alkyles ont de 1 à 5 atomes de carbone;
- Y est un atome d'hydrogène, d'halogène, un groupe cyano, alkyle, alkylthio, haloalkyle, haloalkylthio, alkylsulfényle, alcoxy, carboalcoxy et haloalcoxy, dans lesquels les groupes alkyles ont de 1 à 5 atomes de carbone;
- n represente 1 ou 2; et
- A représente un atome d'hydrogène ou un groupe alkyle inférieur ayant 1 à 3 atomes de carbone; ou un sel de celle-ci efficace du point de vue herbicide et un diluant ou un support acceptable du point de vue agricole.

2. Une composition suivant la revendication 1, caractérisée en ce que dans le cas d'un sel d'iminooxolidine, la partie formant un sel est HX', où X' représente un atome d'halogène.

3. Une composition suivant la revendication 1 ou la revendication 2, caractérisée en ce que dans l'iminooxazolidine, A représente un groupe éthyle.

4. Une composition suivant l'une quelconque des revendications 1 à 3, caractérisée en ce que dans l'iminooxazolidine, X représente un groupe 3-trifluorométhyle; Y représente un atome 4-chloro ou 4-fluoro; et A représente un groupe éthyle.

5. Un procédé pour la production d'une composition herbicide suivant l'une quelconque des revendications 1 à 4, caractérisé en ce qu'il comprend le mélange d'un composé tel que défini dans l'une quelconque des revendications 1 à 4 avec un support ou un diluant acceptable du point de vue agricole.

6. Une méthode pour contrôler des mauvaises herbes indésirables, caractérisée en ce qu'elle comprend l'application sur un site devant être contrôlé, d'une quantité efficace du point de vue herbicide d'une composition suivant l'une quelconque des revendications 1 a 4 ou d'un composé tel que défini dans l'une quelconque des revendications 1 à 4.

7. Un procédé pour la préparation d'un composé tel que défini dans l'une quelconque des revendications 1 à 4, caractérisé en ce qu'il comprend:

(a) la réaction d'un urée alcool correspondant à la formule générale suivante:

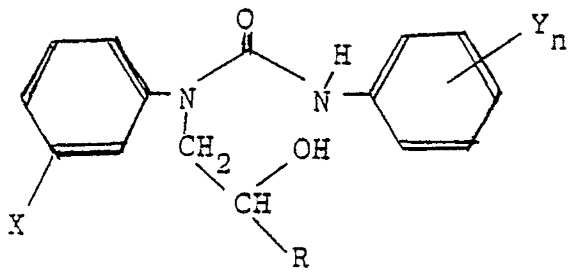

dans laquelle X,Y, n et A sont tels que définis plus haut, avec un agent déshydratant pour former un sel correspondant à la formule générale suivante:

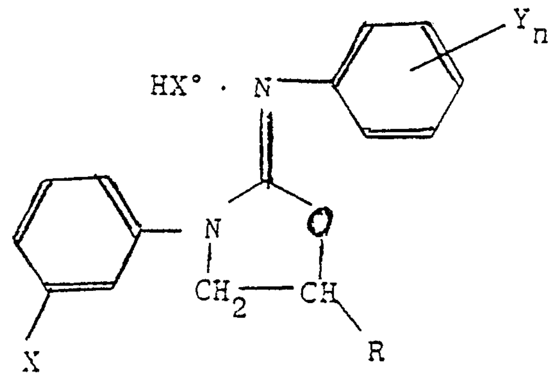

dans laquelle X, Y, n et A sont tels que définis plus haut et X' représente un anion formant un sel; et, éventuellement,

(b) la réaction du sel avec une base pour former un composé correspondant à la formule générale:

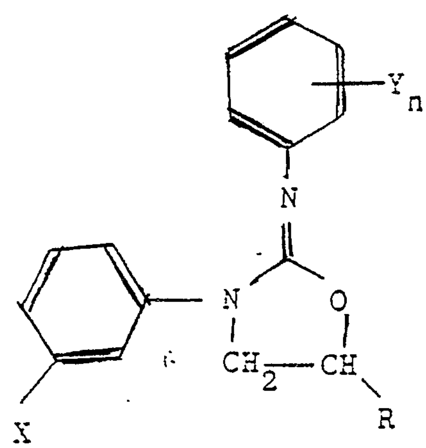

dans laquelle n, X, Y et A sont tels que définis plus haut.

8. Un procédé suivant la revendication 7, caractérisé en ce que l'agent déshydratant est choisi parmi le chlorure de thionyle, le phosgène, l'oxychlorure de phosphore et le pentoxyde de phosphore.

9. Un procédé suivant la revendication 7 ou la revendication 8, caractérisé en ce que la base est choisie parmi la pyridine, la triéthylamine, le carbonate acide de sodium, l'hydroxyde de odium et l'hydroxyde de potassium.

**Ansprüche**

1. Iminooxazolidin,
dadurch **gekennzeichnet,**
daß es der nachstehenden allgemeinen Formel entspricht:

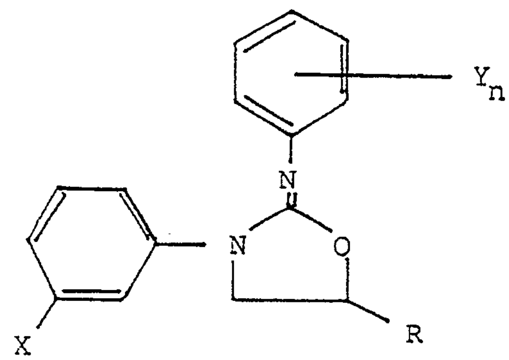

worin
X für Cyano, Halogen, Alkyl, Alkylthio, Haloalkyl, Haloalkylthio, Alkylsulfenyl, Alkoxy, Carboalkoxy und Haloalkoxy steht, wobei die Alkylgruppen 1 bis 5 Kohlenstoffatome besitzen; Y für Wasserstoff, Cyano, Halogen, Alkyl, Alkylthio, Haloalkyl, Haloalkylthio, Alkylsulfenyl, Alkoxy, Carboalkoxy und Haloalkoxy steht, wobei die Alkylgruppen 1 bis 5 Kohlenstoffatome besitzen; n für 1 oder 2 steht; und R für Wasserstoff oder eine Niedrigalkylgruppe mit 1 bis 3 Kohlenstoffatomen steht;
und ein herbizidal wirksames Salz davon.

2. Iminooxazolidinsalz nach Anspruch 1, bei dem $HX^{\circ}$ die salzbildende Einheit darstellt, wobei $X^{\circ}$ für Halogen steht.

3. Verbindung nach Anspruch 1 oder 2, bei der R für Ethyl steht.

4. Verbindung nach einem der Ansprüche 1 bis 3, bei der X für 3-Trifluormethyl steht; Y für 4-Chlor oder 4-Fluor steht; und R für Ethyl steht.

5. Herbizidzusammensetzung,
dadurch **gekennzeichnet,**
daß sie eine Verbindung nach einem der Ansprüche 1 bis 4 und ein(en) landwirtschaftlich verträglichen(s) Träger oder Verdünnungsmittel enthält.

6. Verfahren zur Kontrolle von unerwünschten Unkrautplagen,
dadurch **gekennzeichnet,**
daß man auf den Locus, den man zu kontrollieren wünscht, eine herbizidal wirksame Menge einer Verbindung nach einem der Ansprüche 1 bis 4 oder eine im Anspruch 5 beanspruchte Zusammensetzung aufbringt.

7. Verfahren zur Herstellung einer Verbindung nach einem der Ansprüche 1 bis 4,
dadurch **gekennzeichnet,**
daß man
(a) einen Harnstoffalkohol der folgenden allgemeinen Formel:

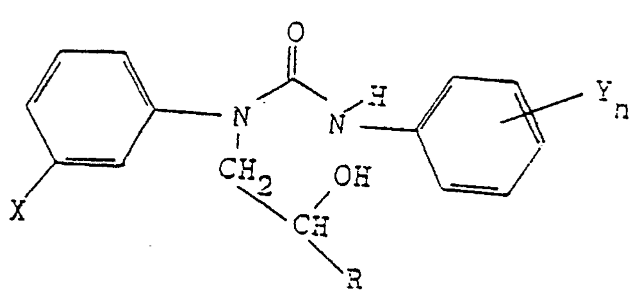

worin X, Y, n und R die oben angegebenen Bedeutungen besitzen, mit einem Dehydratisierungsmittel zu einem Salz der folgenden allgemeinen Formel:

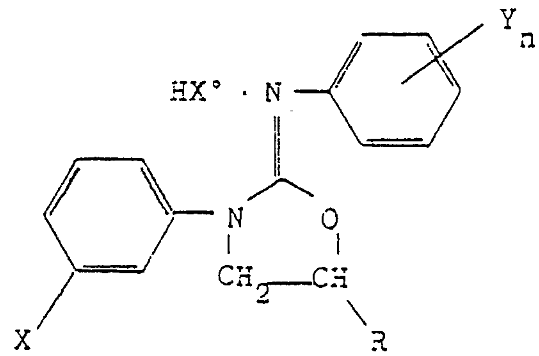

worin x, Y, n und R die oben angegebenen Bedeutungen besitzen und $X^{\circ}$ ein salzbildendes Anion darstellt, umsetzt; und gewünschtenfalls

(b) dieses Salz mit einer Base zu einer Verbindung der folgenden allgemeinen Formel:

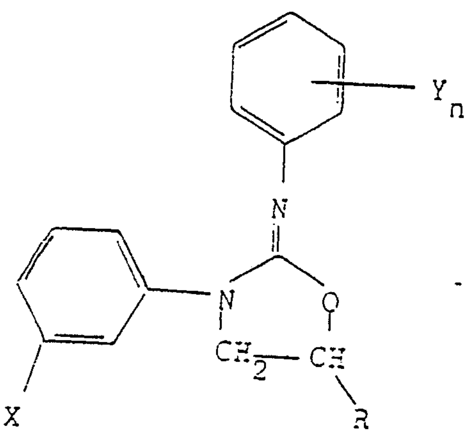

worin n, x, y und R die oben angegebenen Bedeutungen besitzen, umsetzt.

8. Verfahren nach Anspruch 7, bei dem das Dehydratisierungsmittel ausgewählt ist unter Thionylchlorid, Phosgen, Phosphoroxychlorid und Phosphorpentoxid.

9. Verfahren nach Anspruch 7 oder 8, bei dem die Base ausgewählt ist unter Pyridin, Triethylamin, Natriumbicarbonat, Natriumhydroxid und Kaliumhydroxid.

Patentansprüche für folgenden Vertragsstaaten: ES, GR.

1. Herbizidzusammensetzung,
dadurch **gekennzeichnet,**
daß sie ein Iminooxazolidin der nachstehenden allgemeinen Formel:

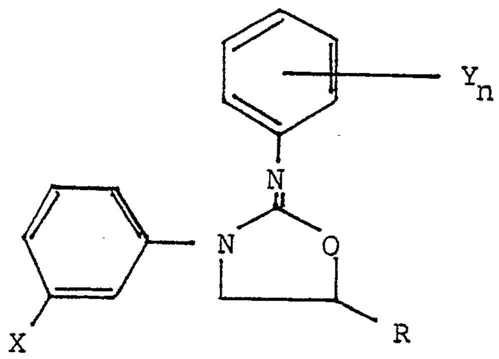

worin
X für Cyano, Halogen, Alkyl, Alkylthio, Haloalkyl, Haloalkylthio, Alkylsulfenyl, Alkoxy, Carboalkoxy und Haloalkoxy steht, wobei die Alkylgruppen 1 bis 5 Kohlenstoffatome besitzen; Y für Wasserstoff, Cyano, Halogen, Alkyl, Alkylthio, Haloalkyl, Haloalkylthio, Alkylsulfenyl, Alkoxy, Carboalkoxy und Haloalkoxy steht, wobei die Alkylgruppen 1 bis 5 Kohlenstoffatome besitzen; n für 1 oder 2 steht; und R für Wasserstoff oder eine Niedrigalkylgruppe mit 1 bis 3 Kohlenstoffatomen steht;
oder ein herbizidal wirksames Salz davon und ein(en) landwirtschaftlich verträglichen(s) Träger oder Verdünnungsmittel enthält.

2. Zusammensetzung nach Anspruch 1, bei der im Falle eines Iminooxazolidinsalzes $HX^{\circ}$ die salzbildende Einheit darstellt, worin $X^{\circ}$ für Halogen steht.

3. Zusammensetzung nach Anspruch 1 oder 2, bei der R im Iminooxazolidin für Ethyl steht.

4. Zusammensetzung nach einem der Ansprüche 1 bis 3, bei der in dem Iminooxazolidin X für 3-Trifluormethyl steht, Y für 4-Chlor oder 4-Fluor steht und R für Ethyl steht.

5. Verfahren zur Herstellung einer Herbizidzusammensetzung nach einem der Ansprüche 1 bis 4,
dadurch **gekennzeichnet,**
daß man eine in einem der Ansprüche 1 bis 4 definierte Verbindung mit einem landwirtschaftlich verträglichen Träger oder Verdünnungsmittel mischt.

6. Verfahren zur Kontrolle von ungewünschten Unkrautplagen,
dadurch **gekennzeichnet,**
daß man auf den Locus, den man zu kontrollieren wünscht, eine herbizidal wirksame Menge einer Zusammensetzung nach einem der Ansprüche 1 bis 4 oder eine in einem der Ansprüche 1 bis 4 definierte Verbindung aufträgt.

7. Verfahren zur Herstellung einer in einem der Ansprüche 1 bis 4 definierten Verbindung,
dadurch **gekennzeichnet,**
daß man
(a) einen Harnstoffalkohol der folgenden allgemeinen Formel:

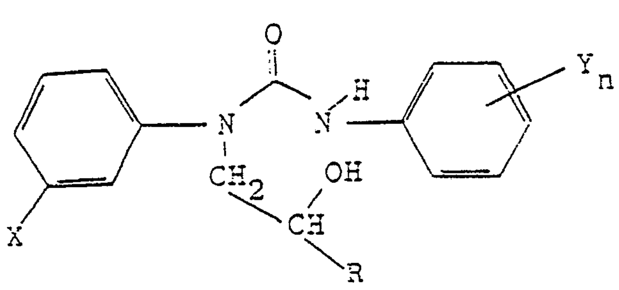

worin X, Y, n und R die oben angegebenen Bedeutungen besitzen, mit einem Dehydratisierungsmittel zu einem Salz der folgenden allgemeinen Formel:

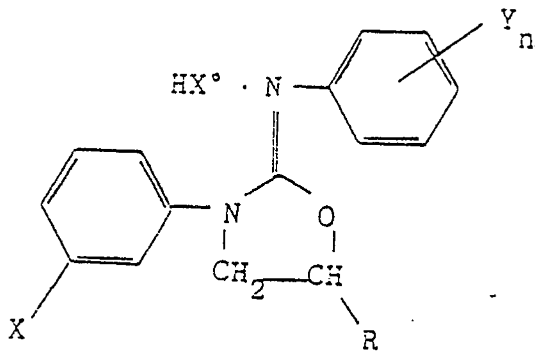

worin X, Y, n und R die oben angegebenen Bedeutungen besitzen und $X^{\circ}$ ein salzbildendes Anion darstellt, umsetzt; und gewünschtenfalls

(b) dieses Salz mit einer Base zu einer Verbindung der folgenden allgemeinen Formel:

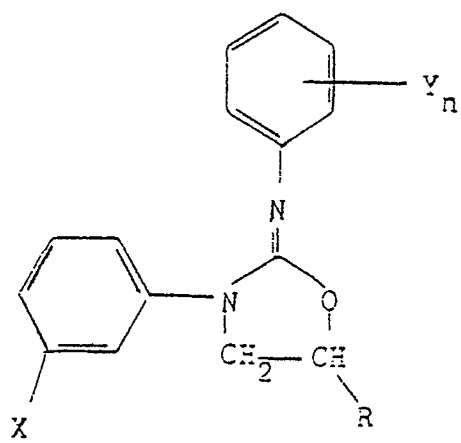

worin n, X, Y und R die oben angegebenen Bedeutungen besitzen, umsetzt.

8. Verfahren nach Anspruch 7, bei dem das Dehydratisierungsmittel ausgewählt ist unter Thionylchlorid, Phosgen, Phosphoroxychlorid und Phosphorpentoxid.

9. Verfahren nach Anspruch 7 oder 8, bei dem die Base ausgewählt ist unter Pyridin, Triethylamin, Natriumbicarbonat, Natriumhydroxid und Kaliumhydroxid.